# EUROPEAN PATENT APPLICATION

(11) **EP 4 459 550 A1**
(43) Date of publication of application: **06.11.2024**
(21) Application number: 22916652.5
(22) Date of filing: 26.12.2022
(51) Int. Cl.: G06T 7/20, G06V 40/20, A63B 24/00

(54) **EXERCISE POSTURE ANALYZING DEVICE AND SMART EXERCISE DEVICE COMPRISING SAME**

(30) Priority: 28.12.2021 KR 20210190353; 22.12.2022 KR 20220182178
(71) Applicant: DRAX Inc., Anyang-si, Gyeonggi-do 14086 (KR)
(72) Inventor: YOO, Seon Kyung, Seoul 08251 (KR)
(74) Representative: Bianchetti & Minoja with Trevisan & Cuonzo IPS SRL
(86) International application number: PCT/KR2022/021298
(87) International publication number: WO 2023/128511

(57) **Abstract**

An exercise posture analyzing device, according to an embodiment, for analyzing an exercise posture of a user of exercise equipment, includes a photographing unit configured to obtain an exercise image of the user by photographing the user who is exercising by using the exercise equipment, an equipment detector configured to detect exercise equipment-related information relating to at least one of a movement and a length of the exercise equipment, and an analyzer configured to calculate a rotation angle of a body part of the user based on the exercise image and identify the exercise posture of the user based on the calculated rotation angle of the body part of the user.

## Description

### Technical Field

The present disclosure relates to an exercise posture analyzing device capable of identifying whether a user exercises while maintaining a proper exercise posture and a smart exercise device including the same.

### Background Art

Exercise equipment is provided in various forms according to body parts targeted to increase muscular strength thereof or purpose of use and may be configured mainly for training of body parts, for example, upper or lower body, by using hands or feet. Users may exercise by moving a selected weight using an exercise structure of exercise equipment.

As users of the exercise equipment may have different body conditions, background knowledge on the exercise, etc., exercise postures of the users of the exercise equipment may vary. However, it may be difficult for the users to check if they are properly using the exercise equipment according to their physical features or athletic abilities, or purpose of the exercise equipment.

### Disclosure

### Technical Problem

According to an embodiment of the present disclosure, an exercise posture analyzing device and a smart exercise device may precisely identify an exercise posture of a user and thus prevent provision of incorrect feedbacks to the user.

### Technical Solution

A device for analyzing an exercise posture of a user using exercise equipment includes a photographing unit configured to obtain an exercise image of the user by photographing the user who is exercising by using the exercise equipment, an equipment detector configured to detect exercise equipment-related information relating to at least one of a movement of the exercise equipment and a length of the exercise equipment, and an analyzer configured to calculate a rotation angle of a body part of the user based on the exercise image and identify an exercise posture of the user based on the calculated rotation angle of the body part.

The analyzer may include a feature point extractor configured to extract a plurality of feature points corresponding a plurality of joints of the user based on the exercise image of the user, an angle calculator configured to generate a plurality of bond lines corresponding to body parts of the user by connecting the plurality of feature points and calculate a rotation angle of at least one bond line of the plurality of bond lines, an angle corrector configured to correct, based on the exercise equipment-related information, the rotation angle calculated by the angle calculator, and a posture identifier configured to identify a degree of deviation of the rotation angle corrected by the angle corrector from a reference rotation angle to identify the exercise posture of the user.

The photographing unit may be a single camera installed to face a side of the exercise equipment.

The exercise equipment may be weight training equipment including a plurality of weight plates and an exercise structure delivering a load of the weight plates to the user, and the equipment detector may include a first sensor configured to directly or indirectly detect a movement distance of the weight plates.

The angle corrector may be configured to calculate at least one of a movement distance and a rotation angle of the exercise structure based on the movement distance of the weight plates detected by the first sensor and correct, based on at least one of the movement distance and an angle change of the exercise structure, the rotation angle calculated by the angle calculator.

The exercise equipment may be barbell exercise equipment including a support bar and weight plates skewered on both ends of the support bar, and the equipment detector may be configured to obtain a length or a movement of the barbell exercise equipment.

The equipment detector may include a second sensor installed in the barbell exercise equipment and configured to detect a movement of the barbell exercise equipment.

The angle corrector may be configured to detect a movement distance of the barbell exercise equipment based on a result detected by the second sensor and correct, based on the movement distance of the barbell exercise equipment, the rotation angle calculated by the angle calculator.

The second sensor may be arranged on the support bar.

The photographing unit may be configured to obtain an image of the support bar by photographing the support bar of the barbell exercise equipment, and the angle corrector may be configured to calculate a length of the support bar based on the image of the support bar and correct, based on the length of the support bar, the rotation angle calculated by the angel calculator.

A smart exercise device according to another embodiment includes exercise equipment and an exercise posture analyzing device configured to analyze an exercise posture of a user of the exercise equipment, wherein the exercise posture analyzing device may include a photographing unit configured to obtain an exercise image of the user by photographing the user who is exercising by using the exercise equipment, an equipment detector configured to detect exercise equipment-related information relating to at least one of a movement of the exercise equipment and a length of the exercise equipment, and an analyzer configured to calculate a rotation angle of a body part of the user during exercise of the user based on the exercise image and identify an exercise posture of the user based on the calculated rotation angle of the body part.

The analyzer may include a feature point extractor configured to extract a plurality of feature points corresponding a plurality of joints of the user based on the exercise image of the user, an angle calculator configured to generate a plurality of bond lines corresponding to body parts of the user by connecting the plurality of feature points and calculate a rotation angle of at least one bond line of the plurality of bond lines, an angle corrector configured to correct, based on the exercise equipment-related information, tge rotation angle calculated by the angle calculator, and a posture identifier configured to identify a degree of deviation of the rotation angle corrected by the angle corrector from a reference rotation angle to identify the exercise posture of the user.

The photographing unit may be a single camera installed to face a side of the exercise equipment.

The exercise equipment may be weight training equipment including a plurality of weight plates and an exercise structure delivering a load of the weight plates to the user, and the equipment detector may include a first sensor configured to directly or indirectly detect a movement distance of the weight plates.

The exercise equipment may be barbell exercise equipment including a support bar and weight plates skewered on both ends of the support bar, and the equipment detector may be configured to obtain a length or a movement of the barbell exercise equipment.

The equipment detector may include a second sensor installed in the barbell exercise equipment and configured to detect a movement of the barbell exercise equipment.

A method of analyzing an exercise posture according to another embodiment includes extracting a plurality of feature points corresponding a plurality of joints of a user based on an exercise image of the user obtained by a photographing unit, generating a plurality of bond lines corresponding to body parts of the user by connecting the plurality of feature points and calculating a rotation angle of at least one bond line of the plurality of bond lines, correcting the calculated rotation angle based on exercise equipment-related information detected by the equipment detector, and identifying a degree of deviation of the corrected rotation angle from a reference rotation angle to identify an exercise posture of the user.

A computer-readable storage medium storing at least one program for executing a method of analyzing an exercise posture according to another embodiment includes instructions for extracting a plurality of feature points corresponding to a plurality of joints of a user based on an exercise image of the user obtained by a photographing unit, instructions for generating a plurality of frames lines corresponding to body parts of the user by connecting the plurality of feature points and calculating a rotation angle of at least one bond line of the plurality of bond lines, instructions for correcting the calculated rotation angle based on exercise equipment-related information detected by an equipment detector; and instructions for identifying a degree of deviation of the corrected rotation angle from a reference angle to identify an exercise posture of the user.

### Advantageous Effects

According to an embodiment of the present disclosure, an exercise posture analyzing device and a smart exercise device including the same may analyze an exercise posture of a user by using an exercise image of the user and provide correct feedbacks on the exercise posture to the user through correction of errors by using a separate tool independent from the user.

### Description of Drawings

FIG. 1 is a block diagram illustrating a smart exercise device according to an embodiment.
FIG. 2 is a perspective view illustrating a portion of a smart exercise device according to an embodiment.
FIGS. 3 and 4 are each a diagram for describing a process by which the smart exercise device of FIG. 2 detects an exercise posture of a user.
FIG. 5 is a diagram for conceptually describing operations of an exercise posture analyzing device according to an embodiment.
FIG. 6 is a diagram for conceptually describing a rotation angle of a bond line illustrated in FIG. 5.
FIGS. 7 and 8 are each a diagram for conceptually describing a rotation angle of a bond line according to various body conditions.
FIG. 9 is a diagram for describing an example of an equipment detector of an exercise posture analyzing device according to an embodiment.
FIG. 10 is a diagram illustrating an example of a user interface unit according to an embodiment.
FIGS. 11 and 12 are each a diagram for describing an exercise posture analyzing device applied to exercise equipment according to another embodiment.
FIG. 13 is a diagram for describing an example of an equipment detector applied to the exercise equipment of FIG. 11.
FIG. 14 is a diagram for describing another example of an equipment detector applied to the exercise equipment of FIG. 11.
FIG. 15 is a diagram for describing an example of an equipment detector according to another embodiment.
FIG. 16 is a block diagram illustrating an exercise posture analyzing device including an equipment detector according to another embodiment.
FIG. 17 is a diagram illustrating a smart gym environment in which a plurality of smart exercise devices are provided according to an embodiment.

### Best Mode

### Mode for Invention

Hereinafter, various embodiment of the present disclosure are described in detail with reference to the attached drawings such that a person of ordinary skill in the pertinent art may easily perform the present disclosure.

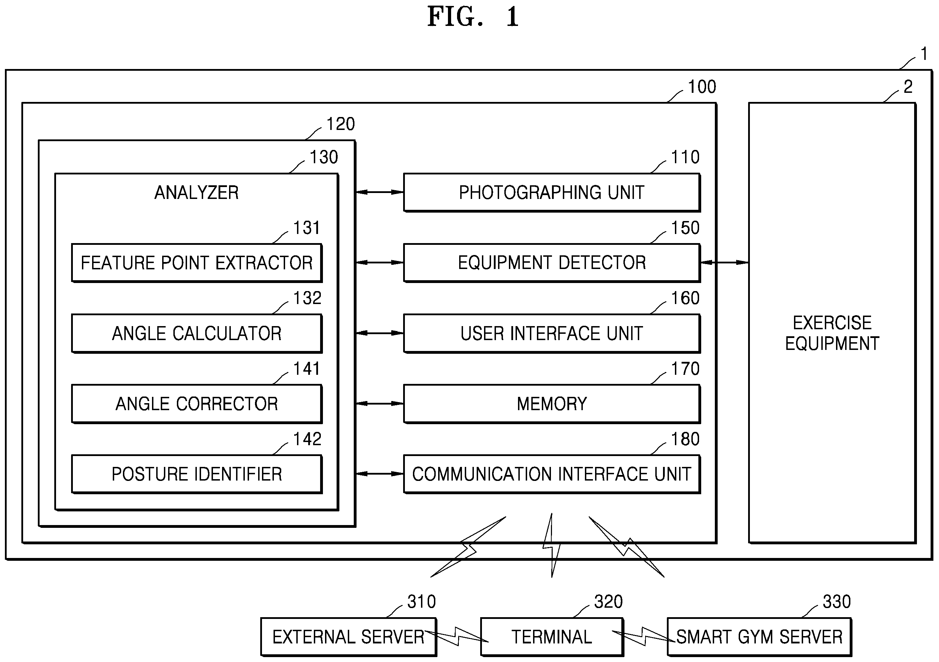

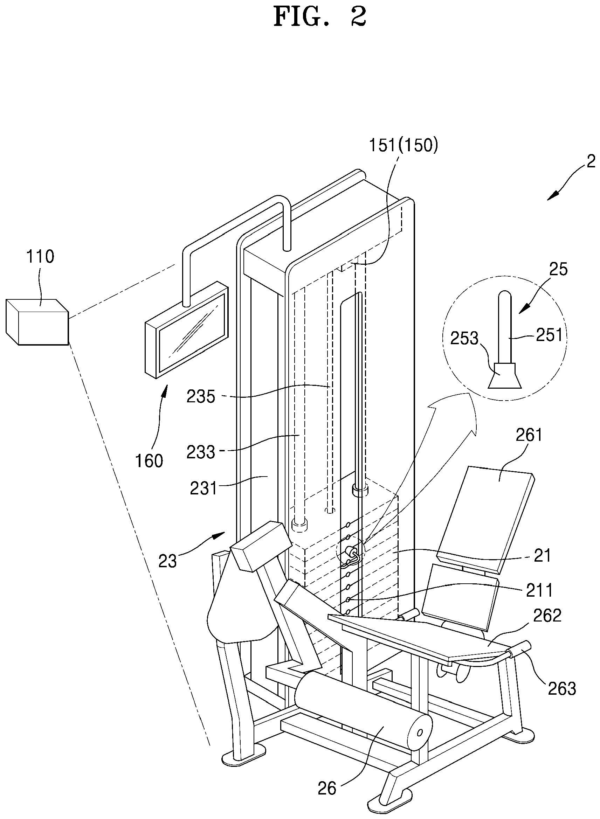

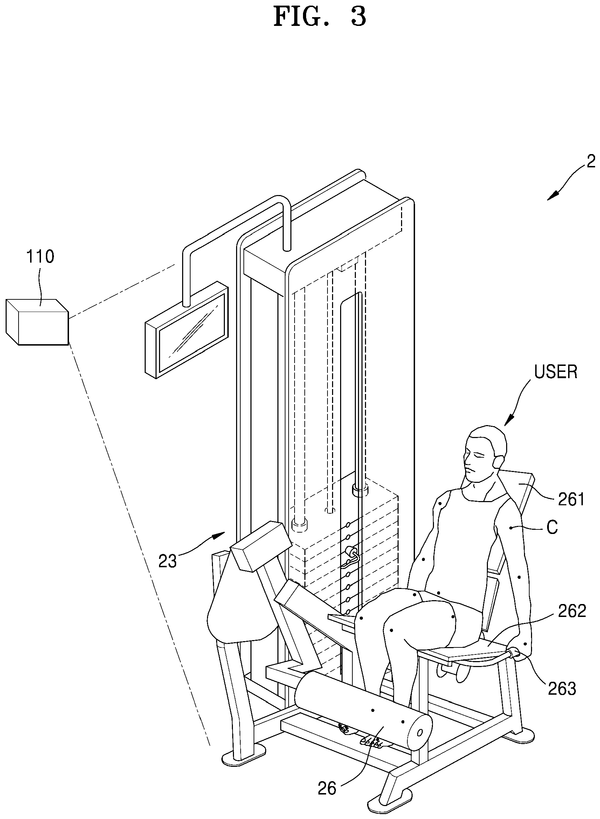

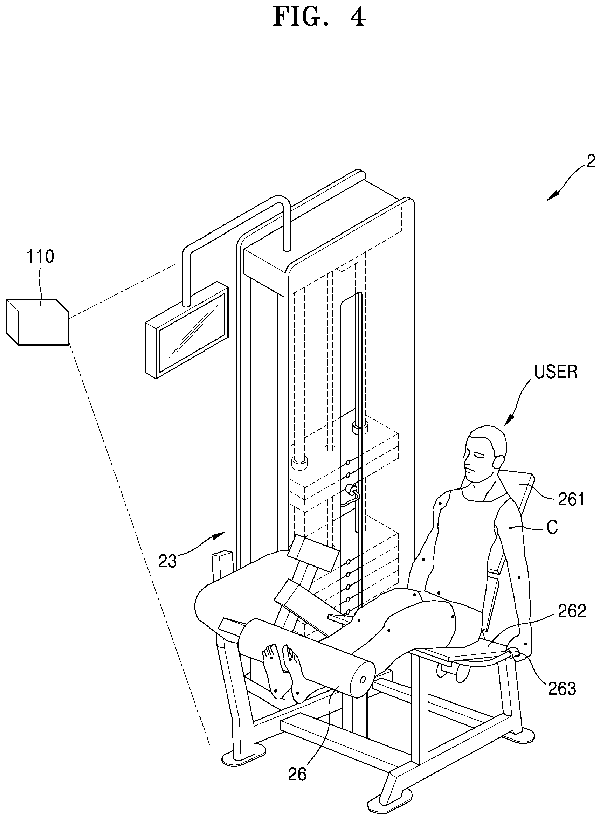

FIG. 1 is a block diagram illustrating a smart exercise device 1 according to an embodiment. FIG. 2 is a perspective view illustrating a portion of the smart exercise device 1 according to an embodiment. FIGS. 3 and 4 are each a diagram for describing a process by which the smart exercise device 1 of FIG. 2 detects an exercise posture of a user USER.

Referring to FIG. 1, the smart exercise device 1 according to an embodiment may include exercise equipment 2 and an exercise posture analyzing device 100.

Referring to FIGS. 1 and 2, the exercise equipment 2 may be exercise equipment in which movements occur according to weight training by the user USER. The exercise equipment 2 may be weight training equipment.

For example, the exercise equipment 2 may include a plurality of weight plates 21, a frame structure 23 supporting the weight plates 21 such that the weight plates 21 may move in the direction of gravity and the opposite direction thereof, for example, the vertical direction, and an exercise structure 26 in contact with a body part of the user USER and delivering a load of the weight plates 21.

The plurality of weight plates 21 may be sequentially stacked in the vertical direction. Each of the plurality of weight plates 21 may have a certain weight. The weights of the weight plates 21 may be identical to or different from each other. For example, the plurality of weight plates 21 may have the same weight of 5 kg. Or, some of the plurality of weight plates 21 may be 5 kg, while others may be 10 kg. In addition to the above, the weight of the plurality of weight plates 21 may vary.

The frame structure 23 may include a base frame 231 and a pair of guide rails 233 extending in the vertical direction and arranged in the base frame 231 such that the plurality of weight plates 21 may move in the vertical direction. The pair of guide rails 233 may be arranged to penetrate the plurality of weight plates 21. The frame structure 23 may include a connection line 235 configured to deliver the force applied by the user USER to the weight plates 21.

When using the exercise equipment 2 according to an embodiment, the user USER may apply forces to the exercise structure 26 to move the weight plates 21 corresponding to a selected weight in the gravity direction or the opposite direction thereof. The exercise structure 26 may be implemented in various forms according to a target body part.

For example, the exercise equipment 2 may be a leg extension. In the exercise structure 26, a pad configured to be in contact with an ankle of the user USER (or a shin and below) may rotate around a certain axis.

Referring to FIGS. 3 and 4, when the exercise equipment 2 is a leg extension, the user USER may sit on a chair of the exercise equipment 2 and put the ankles into contact with the exercise structure 26. Then, by performing a reciprocating motion of bending and straightening the knees, thigh muscles of the user USER may be stimulated.

However, the effects of such exercise may be achieved only when the user performs the exercise in a reference posture that suits the purpose of the exercise structure 26. For example, the user USER may achieve desired exercise effects when performing the exercise in the reference posture of the reciprocating motion of bending and straightening the knees while maintaining the angle of 90 degrees between the shin and the feet and being seated on a chair with the back of the user USER touching a back 261 of the chair and the both hands holding handles 263 such that the hips remain attached to a seat 262 of the chair.

However, when the users USER exercise alone using the exercise equipment 2 without help of a trainer, as the users USER may have different body conditions, background knowledge about the exercise, etc., some of the users USER using the exercise equipment 2 may exercise in an incorrect posture different from the reference posture.

Even when the number of weight plates 21 set in the exercise equipment 2 is the same, and the exercise structure 26 moves along a fixed track, the exercise effects may vary according to an exercise posture of the user USER. In other words, even when the weight and movement distance of the weight plates 21 are the same, the loads actually applied to a target body part of the user USER, for example, thighs may be different.

Moreover, when the exercise posture of the user USER is different from the reference posture, the load of the exercise equipment 2 may be applied to other body parts of the user USER other than an intended body part, which may lead to an injury to the user USER. For example, when the user USER exercises in an incorrect posture, the load may be applied to the knees instead of thighs, which may result in a knee injury to the user USER. The greater the set number of weight plates 21 is, the higher the risk of injury to the user USER may be.

Considering the above, the exercise posture analyzing device 100 according to an embodiment may analyze whether the user USER exercise in an exercise posture corresponding to the reference posture. For example, the exercise posture analyzing device 100 may include a photographing unit 110 and an analyzer 120.

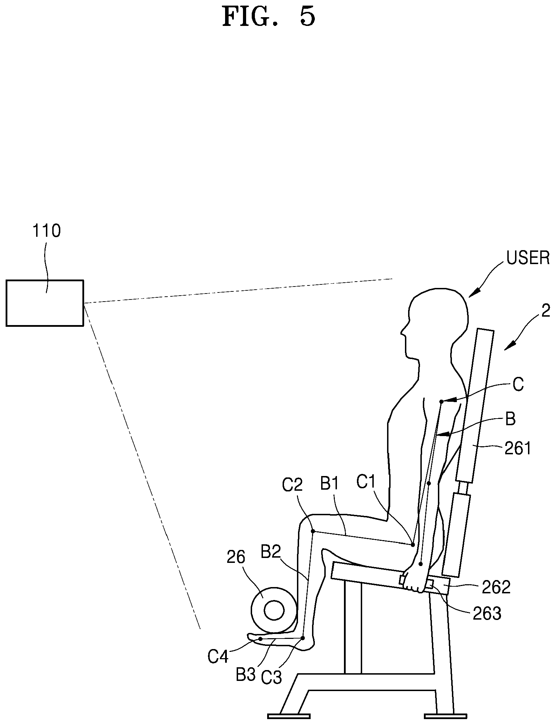

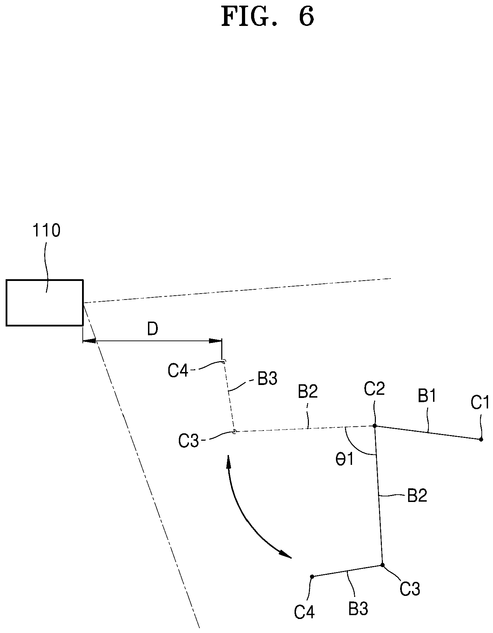

FIG. 5 is a diagram for conceptually describing operations of the exercise posture analyzing device 100 according to an embodiment, and FIG. 6 is a diagram for conceptually describing a rotation angle of a bond line B illustrated in FIG. 5.

Referring to FIGS. 2 to 5, the photographing unit 110 may obtain an exercise image of the user USER by photographing the user USER who is exercising by using the exercise equipment 2.

The photographing unit 110 may be arranged to measure the exercise of the user USER using the exercise equipment 2. For example, the photographing unit 110 may be arranged on one side of the exercise equipment 2. For example, the photographing unit 110 may be arranged on the front of the exercise equipment 2. The photographing unit 110 may be arranged on a lateral side of the exercise equipment 2.

The photographing unit 110 may consecutively photograph exercise images of the user USER who is exercising by using the exercise equipment 2. The photographing unit 110 may consecutively obtain n image frames. Through this, exercise images of the user USER who is exercising by using the exercise equipment 2 may be obtained consecutively.

The photographing unit 110 may be a single camera. The photographing unit 110 may be spaced apart from the user USER or the exercise equipment 2 at a certain distance D. As the photographing is performed by using a single camera, the unit price may be lowered, compared to the photographing unit 110 using multiple cameras, and accordingly, the exercise posture analyzing device 100 may have a competitive price.

The analyzer 120 may calculate a rotation angle of a body part during the exercise of the user USER based on the exercise image. The analyzer 120 may identify an exercise posture of the user USER based on the calculated rotation angle of the body part. To this end, the analyzer 120 may include a feature point extractor 120, an angle calculator 132, an angle corrector 141, and a posture identifier 142.

The feature point extractor 120 may extract a plurality of feature points C corresponding to a plurality of joints of the user USER based on the exercise image of the user USER. For example, the feature point extractor 120 may extract feature points C corresponding to a shoulder, an elbow, a wrist, a pelvis, a knee, an ankle, and a foot of the user USER. As the extraction process of the feature points C itself is well known in the field, specific description thereon is omitted.

Referring to FIGS. 1, 5, and 6, the angle calculator 132 may generate the bond line B connecting the plurality of feature points C extracted by the feature point extractor 120. For example, a first bond line B1 connecting a first feature point C1 corresponding to a pelvis to a second feature point C2 corresponding to a knee, a second bond line B2 connecting the second feature point C2 corresponding to a knee to a third feature point C3 corresponding to an ankle, and a third bond line B3 connecting the third feature point C3 corresponding to an ankle to a fourth feature point C4 corresponding to a foot may be generated.

Referring to FIGS. 1 to 6, the angle calculator 132 may calculate a rotation angle of at least one bond line B of a plurality of bond lines B during the exercise of the user USER. For example, the angle calculator 132 may calculate a rotation angle θ1 of the second bond line B2 with respect to the first bond line B1. For example, the angle calculator 132 may calculate a rotation angle of the third bond line B3 with respect to the second bond line B2.

Under ideal conditions, the rotation angle (or change in angle) calculated by the angle calculator 132 may accurately match an actual rotation angle of a body part of the user USER. However, under actual conditions, due to various factors such as the photographing unit 110 using a single camera, different body conditions, etc., the rotation angle of the bond line B calculated by the angle calculator 132 may be different from the actual rotation angle of the body part of the user USER.

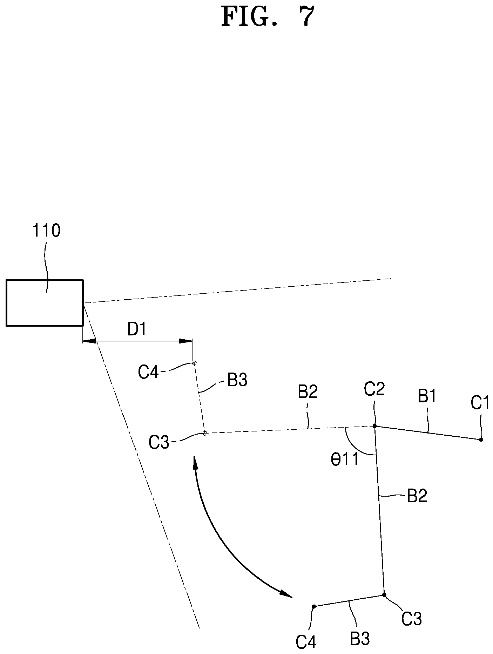

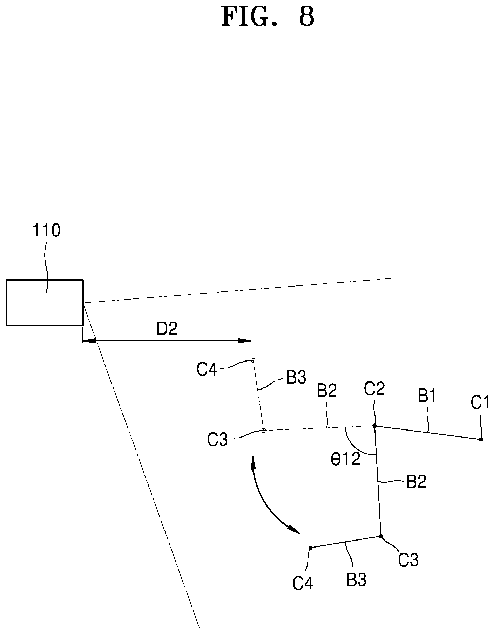

For example, when the photographing unit 110 photographs the exercise of the user USER by using a single camera, errors may occur in the process of extracting the feature points C corresponding to joints of the user USER having a three-dimensional (3D) structure. For example, as illustrated in FIGS. 7 and 8, as users USER may have different body conditions from each other, a distance (D1 and D2) between the user USER and the photographing unit 110 may vary. In the process of extracting the feature points C, there may be a discrepancy between the feature points C and actual positions of the joints of the user USER, and during the generation of bond line B based on the feature points C, there may also be errors in the actual positions of the body parts of the user USER. Accordingly, there may be a discrepancy between the rotation angle calculated by the angle calculator 132 and the actual rotation angle of the body part of the user USER.

As such, when the rotation angle calculated by the angle calculator 132 is different from the actual rotation angle of the body part of the user USER, it may be difficult to accurately identify whether the exercise posture of the user USER is in accordance with the reference posture. In other words, there may be incorrect identification as to whether the exercise posture of the user USER is in accordance with the reference posture. For example, the exercise posture of the user USER may be identified as a correct posture in accordance with the reference posture even when it is an incorrect posture which does not correspond to the reference posture. Or, the exercise posture of the user USER may be identified as an incorrect posture which does not correspond to the reference posture, even when it is a correct posture in accordance with the reference posture.

When such incorrect identification is repeated, the reliability of the exercise posture analyzing device 100 may decrease, and such lowered reliability may serve as a critical reason why the user USER may not use the exercise posture analyzing device 100.

Referring back to FIG. 1, the exercise posture analyzing device 100 according to an embodiment may include an equipment detector 150, the angle corrector 141, and the posture identifier 142 to provide accurate feedbacks on exercise posture of the user USER.

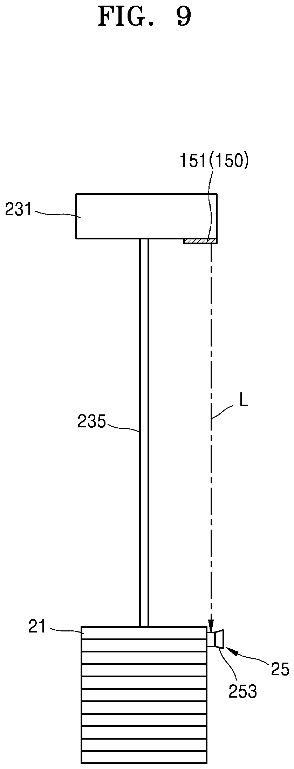

The equipment detector 150 may obtain exercise equipment 2-related information associated with at least one of a movement and a length of the exercise equipment 2. For example, the equipment detector 150 may obtain information associated with the movement of the exercise equipment 2. Detailed description of the equipment detector 150 is to be provided in relation to FIG. 9.

The angle corrector 141 may correct, based on the exercise equipment 2-related information, a rotation angle calculated by the angle calculator 132. The angle corrector 141 may correct, based on information independent from image information of the photographing unit 110, errors in the rotation angle calculated based on the exercise image photographed by the photographing unit 110.

FIG. 9 is a diagram for describing an example of the equipment detector 150 of the exercise posture analyzing device 100 according to an embodiment.

Referring to FIGS. 1, 2, and 9, the equipment detector 150 according to an embodiment may include a first sensor 151 configured to detect movement of the weight plates 21. The first sensor 151 may directly detect the movement of the weight plates 21 or may indirectly detect the movement of the weight plates 21 by detecting the movement of other components moving with the weight plates 21.

For example, the first sensor 151 may include at least one laser sensor. For example, the first sensor 151 may be arranged to detect movement of a pin structure 25. The pin structure 25 may include an insert area 251 inserted into the weight plates 21 or between the weight plates 21 and a holder area 253 connected to the insert area 251. For example, the first sensor 151 may be arranged to irradiate a laser beam L to the holder area 253 of the pin structure 25. For example, the first sensor 151 may be overlap with the holder area 253 in the gravity direction. The first sensor 151 may indirectly detect the movement of the weight plates 21 by detecting the movement of the pin structure 25. However, the target object and the arrangement of the first sensor 151 is not limited thereto and may vary when necessary.

The first sensor 151 may detect a movement distance of the weight plates 21. The angle corrector 141 may calculate at least one of a movement distance and a rotation angle of the exercise structure 26 based on the movement distance of the weight plates 21. For example, when the exercise structure 26 has a rotatable structure, the rotation angle of the exercise structure 26 may be calculated based on the movement distance of the weight plates 21. Or, although it is not shown in the drawings, when the exercise structure 26 has a structure moving linearly, the movement distance of linear movement of the exercise structure 26 may be calculated based on the movement distance of the weight plates 21.

The angle corrector 141 may calculate the rotation angle of the body part of the user USER in contact with the exercise structure 26 based on the rotation angle of the exercise structure 26. For example, based on the information detected by the first sensor 151 which is independent from the photographing unit 110, the rotation angle of the ankle or the shin of the user USER in contact with the exercise structure 26 may be calculated during the exercise.

The angle corrector 141 may only calculate the rotation angle of some body parts of the user USER based on the information detected by the first sensor 151, and it may be difficult to calculate the rotation angle of other body parts of the user USER by the angle corrector 141. For example, the angle corrector 141 may only calculate the rotation angle of the shin of the user USER based on the information calculated by the first sensor 151 and may not calculate the posture of other body parts of the user USER.

However, the rotation angle calculated based on the information detected by the first sensor 151 may be relatively accurate, compared to the rotation angle calculated based on the exercise image photographed by the photographing unit 110. In other words, the rotation angle calculated based on the information detected by the first sensor 151 may have relatively less errors, compared to the rotation angle calculated based on the exercise image photographed by the photographing unit 110. That is, the rotation angle calculated based on the information detected by the first sensor 151 may cover less body parts than the rotation angle calculated based on the information detected by the photographing unit 110 but may have a higher measurement accuracy.

Considering such difference in accuracy, the angle corrector 141 may calculate errors in the rotation angle calculated by the angle calculator 132 by using the rotation angle calculated based on the information detected by the first sensor 151. The angle corrector 141 may correct the rotation angle calculated by the angle calculator 132 such that the calculated errors are reflected.

For example, when there is an error between the rotation angle of the body part calculated based on the first sensor 151 and the rotation angle of the body part calculated based on the photographing unit 110, the overall positions of the feature points C may be modified to positions at which such errors are corrected. For example, the positions of the first feature point C1, the second feature point C2, and the third feature point C3 may be modified to reflect the error between the rotation angle of the shin calculated based on the photographing unit 110 and the rotation angle of the shin calculated based on the first sensor 151. When modifying the positions of the first feature point C1, the second feature point C2, and the third feature point C3, positions of the other feature points C, for example, the position of the fourth feature point C4 may also be modified. The position of the fourth feature point C4 may be modified to correspond to the modified distances and ratios of the first feature point C1, the second feature point C2, and the third feature point C3. By such modification of the positions of the feature points C, not only the bond line B corresponding to the body part in relation to which the errors are detected, but also at least one of a distance and an angle of the bond line B corresponding to another body part in relation to which no error is detected may be modified. For example, based on the information detected by the first sensor 151, both of the length of the second bond line B2 corresponding to the shin and the length of the third bond line B3 corresponding to the foot may be modified.

The posture identifier 142 may identify a degree of deviation of rotation angle based on a reference rotation angle corrected by the angle corrector 141 to identify the exercise posture of the user USER.

The reference rotation angle may be set in various manners. For example, the reference rotation angle may be determined based on an exercise image of a reference posture of a trainer using the exercise equipment 2. For example, a plurality of feature points C may be extracted from the exercise image of the trainer, and based on a rotation angle of the bond line B connecting the plurality of feature points C, the reference rotation angle may be determined.

The posture identifier 142 may compare the corrected rotation angle of the bond line B with the reference rotation angle. Whether the corrected rotation angle and the reference rotation angle are out of a preset reference range may be identified. From this, a degree of deviation of the exercise posture of the user USER from the reference posture may be identified.

Based on the results of identification by the posture identifier 142, the user USER may be provided with feedbacks on whether the exercise posture is correct.

For example, when it is determined that the exercise posture of the user USER matches the reference posture within the reference range, the user USER may be informed that the exercise posture is correct. For example, when it is determined that the exercise posture of the user USER does not match the reference posture and out of the reference range, the user USER may be informed that the exercise posture is incorrect.

A method of analyzing an exercise posture including the following operations may be performed by the exercise posture analyzing device 100. For example, the method of analyzing an exercise posture may include: extracting a plurality of feature points C corresponding to a plurality of joints of the user USER based on an exercise image of the user USER obtained by the photographing unit 110; generating a plurality of bond lines B corresponding to body parts of the user USER by connecting the plurality of feature points C and calculating a rotation angle of at least one bond line B from among the plurality of bond lines B; correcting the calculated rotation angle based on exercise equipment 2-related information detected by the equipment detector 150; and identifying a degree of deviation of the corrected rotation angle from a reference rotation angle to identify an exercise posture of the user USER.

Referring back to FIG. 1, the exercise posture analyzing device 100 according to an embodiment may further include a component for measuring an exercise state of the user USER and providing the result in the exercise equipment 2.

For example, the exercise posture analyzing device 100 may include the equipment detector 150, a user interface unit 160 configured to output a user interface screen, a memory 170 configured to store at least one instruction, and a processor 120 configured to control the user interface unit 160.

The equipment detector 150 may perform the function of detecting an exercise state of the user USER in addition to the function of supplementing the accuracy during the analysis of the exercise posture of the user USER. For example, the equipment detector 150 may detect an exercise speed of the user USER by detecting a movement distance and a movement speed of the weight plates 21.

The user interface unit 160 may include an input unit configured to receive an input for operation of the exercise equipment 2, an input for setting, etc. from the user USER and an output unit configured to display information such as an exercise state, an exercise result, etc. For example, the user interface unit 160 may be in the form of a touch screen; however, the disclosure is not limited thereto.

The processor 120 may manage information for managing various functions provided by the smart exercise device 1 or exercise states of the user USER by executing at least one instruction stored in the memory 170. The exercise states of the user USER may include a count or time of exercise, an exercise level, an exercise speed, a trace of body parts, etc. of the user USER. The processor 120 may include the analyzer 120.

The processor 120 may include at least one processing module. For example, the processor 120 may include at least one of a central processing unit, a microprocessor, a graphic processing unit, an application specific integrated circuit (ASIC), a digital signal processor (DSP), and a field programmable gate array (FPGA). The processor 120 may control other components included in the exercise equipment 2 to perform a function corresponding to an input by the user USER received through the user interface unit 160. The processor 120 may execute instructions, a software module, or a program stored in the memory 170, read data or files stored in the memory 170, or store a new program or an application in the memory 170.

The memory 170 may store at least one instruction. The processor 120 may be a computer capable of executing instructions stored in the memory 170. The memory 170 may store instructions, a software module, or a program. The memory 170 may include at least one of a random access memory (RAM), a static random access memory (SRAM), a read-only memory (ROM), a flash memory, an electrically erasable programmable read-only memory (EEPROM), a programmable read-only memory (PROM), a magnetic memory, a magnetic disk, and an optical disk.

A user interface (UI) module and an exercise management module may be stored in the memory 170. The user interface module and the exercise management module may each be a software module or program including at least one instruction or may be a part of another program. The processor 120 may call the user interface module and the exercise management module from the memory 170 and execute the instructions.

The user interface module may include a user interface input/output module and a user interface configuration module. The user interface input/output module may identify an input by the user USER through a user interface screen displayed on the user interface unit 160 and control an output of a user interface element generated or changed by the user interface configuration module. The user interface configuration module may generate or change a user interface element to be displayed on the user interface unit 160 according to information confirmed through the exercise management module, the user interface unit 160, a sensor module, etc.

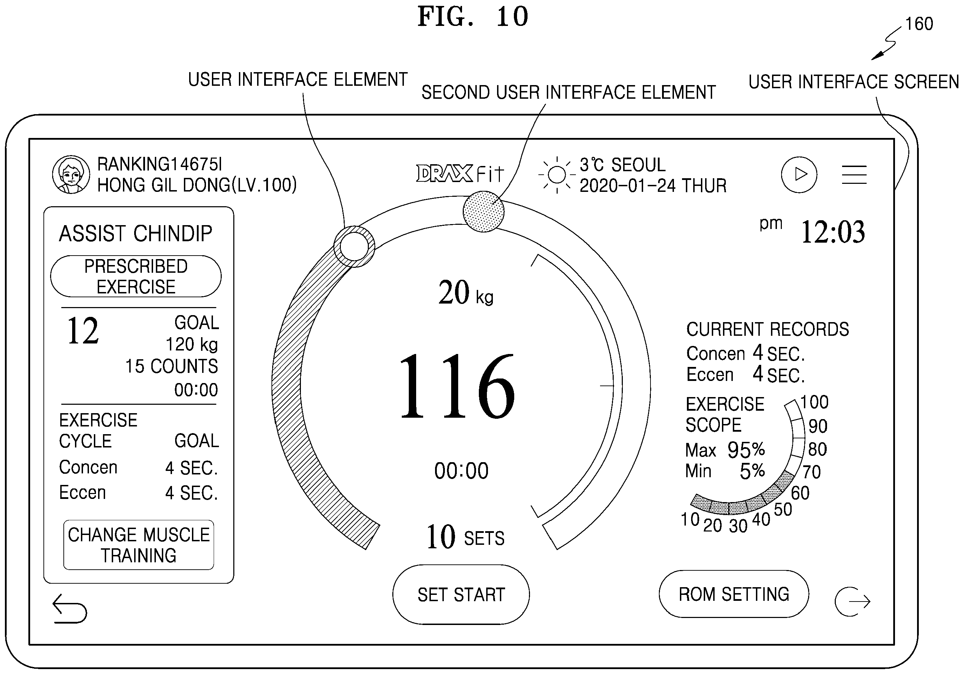

FIG. 10 is a diagram illustrating an example of the user interface unit 160 according to an embodiment.

Referring to FIGS. 1, 9, and 10, the processor 120 may control the user interface unit 160 to display a user interface element showing an exercise state of the user USER corresponding to a movement of the exercise equipment 2 detected by the equipment detector 150 on the user interface screen. The processor 120 may control the user interface unit 160 to display a second user interface element showing an exercise guide recommended for exercise using the exercise equipment 2 along with the user interface element on the user interface screen.

As such, the user USER using the exercise equipment 2 may recognize a weight setting state and an exercise state by using data (or information) displayed on the user interface screen. Through this, the user USER may exercise more effectively.

The exercise posture analyzing device 100 may further include a communication interface unit 180. The communication interface unit 180 may perform wired/wireless communication with other devices or a network. To this end, the communication interface unit 180 may include a communication module supporting at least one of various wired/wireless communication methods. For example, a communication module configured to perform short range communication such as wireless fidelity (Wi-Fi), various types of mobile communication such as 3G, 4G, 5G, etc., or ultra wideband communication or a communication module configured to perform wired communication using a coaxial cable, an optical fiber cable, etc. may be included. However, the present disclosure is not limited thereto, and various other types of communication module according to development of communication technology may be included. The communication interface unit 180 may be connected to a device located outside the exercise equipment 2 and may receive and transmit a message including a signal or data. The exercise posture analyzing device 100 may perform communication with a smart gym server 200, a terminal 320, such as a wearable device or a smartphone, an administrator terminal 300, such as a personal computer (PC), a laptop, or a smartphone, or an external server 310 through the communication interface unit 180.

The foregoing embodiments are described focusing on weight training equipment including the plurality of weight plates 21 as an example of the exercise equipment 2; however, application of the exercise posture analyzing device 100 is not limited thereto, and the exercise posture analyzing device 100 may be applied to various exercise equipment 2.

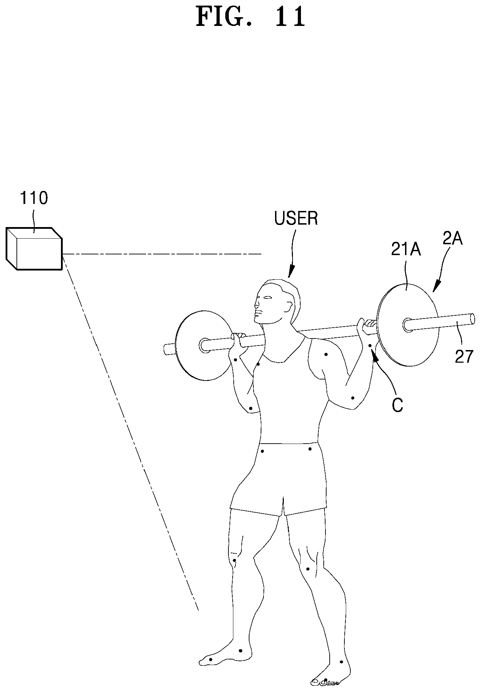

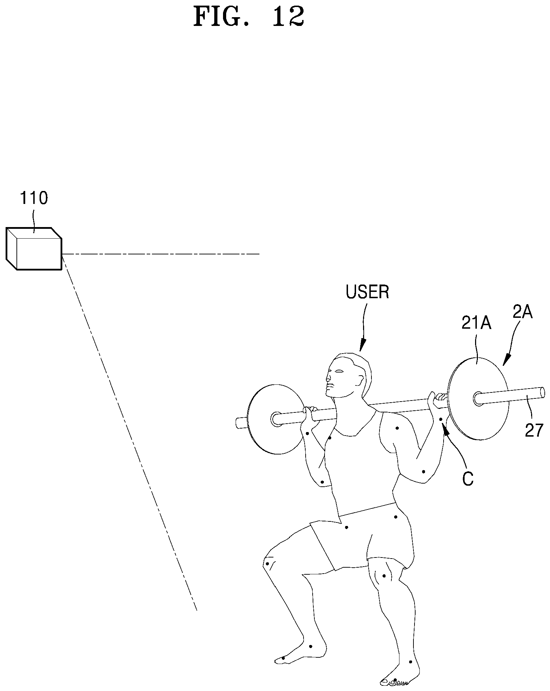

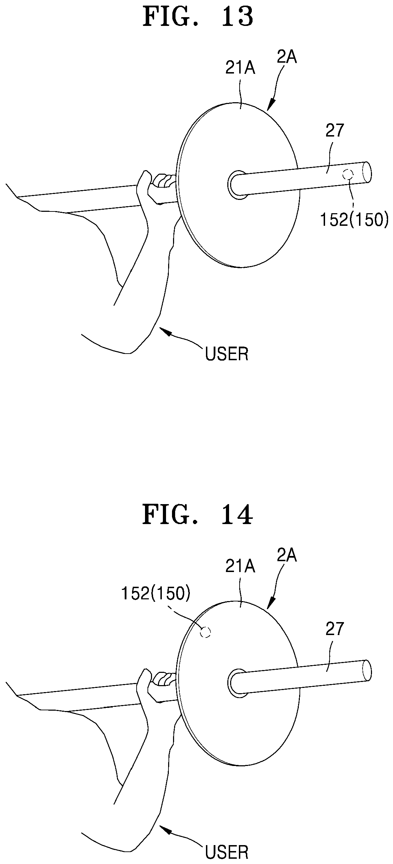

FIGS. 11 and 12 are each a diagram for describing the exercise posture analyzing device 100 applied to exercise equipment 2A according to another embodiment. FIG. 13 is a diagram for describing an example of the equipment detector 150 applied to the exercise equipment 2A of FIG. 11. FIG. 14 is a diagram for describing another example of the equipment detector 150 applied to the exercise equipment 2A of FIG. 11.

Referring to FIGS. 1, 11, and 12, the exercise equipment 2A may be barbell exercise equipment. For example, the exercise equipment 2A may include a support bar 27 having both ends on which a plurality of weight plates 21A may be skewered.

When the user USER wishes to perform lower body exercise using the exercise equipment 2A according to an embodiment, the user USER may put the support bar 27 securely on the shoulder and then bend and straighten the knees while maintaining the back straight.

The exercise posture analyzing device 100 according to an embodiment may include the photographing unit 110, the equipment detector 150, and the analyzer 120. The analyzer 120 may include the feature point extractor 120, the angle calculator 132, the angle corrector 141, and the posture identifier 142. As the photographing unit 110 and the analyzer 120 have already been described, embodiments are now described focusing on the differences, i.e., the equipment detector 150.

When the exercise equipment 2A includes barbell exercise equipment, the equipment detector 150 may include a second sensor 152 arranged on the barbell exercise equipment. The second sensor 152 may be configured to detect movement of the barbell exercise equipment.

The second sensor 152 may be at least one of an acceleration sensor and a gyro sensor. However, the second sensor 152 is not limited thereto, and various modifications may be made to the extent that the movement of the barbell exercise equipment is detected.

Referring to FIG. 13, the second sensor 152 may be arranged on the support bar 27. Accordingly, a movement distance of the support bar 27 may be detected during the exercise of the user USER. Based on the detected movement distance of the support bar 27, a rotation angle of the shin of the user USER may be estimated.

Although FIG. 13 illustrates that the second sensor 152 is arranged on the support bar 27, the arrangement of the second sensor 152 is not limited thereto and may vary. For example, as illustrated in FIG. 14, the second sensor 152 may be arranged on each of the weight plates 21A.

Although the embodiment describe that the first sensor 151 or the second sensor 152 as one of components of the equipment detector 150, the equipment detector 150 may not include a separate sensor.

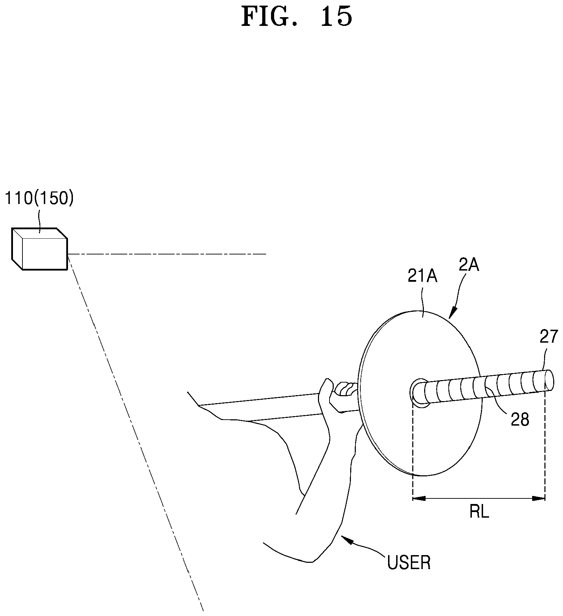

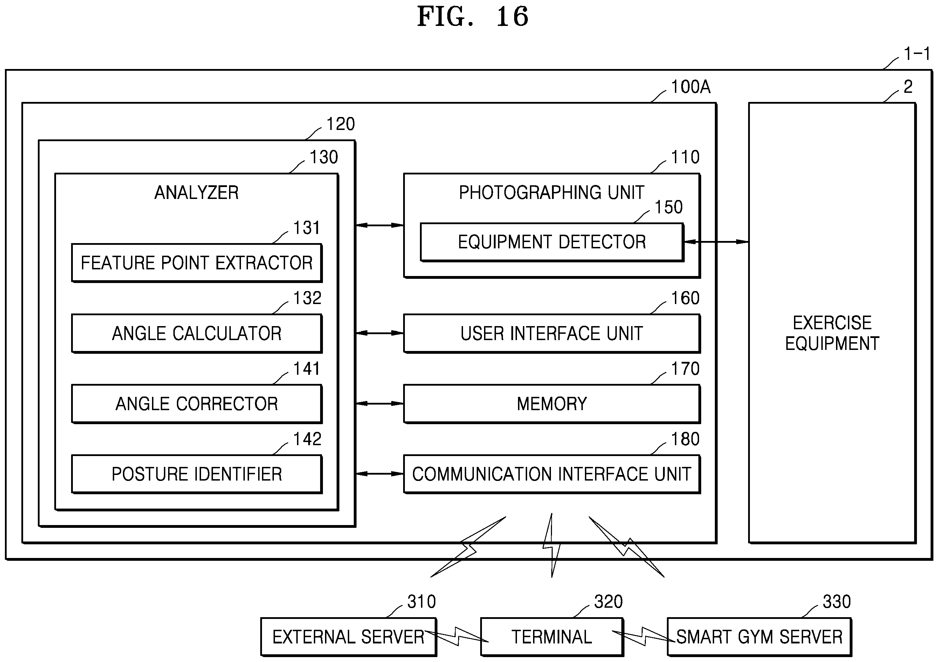

FIG. 15 is a diagram for describing an example of the equipment detector 150 according to another embodiment, and FIG. 16 is a block diagram illustrating an exercise posture analyzing device 100A including the equipment detector 150 according to another embodiment.

Referring to FIGS. 15 and 16, a smart exercise device 1-1 according to an embodiment may include the exercise posture analyzing device 100A and the exercise equipment 2.

The equipment detector 150 may not include a separate sensor and may obtain information related to the exercise equipment 2 based on information about peripheral components other than body parts of the user USER from among information photographed by the photographing unit 110.

For example, the equipment detector 150 may detect the length of the support bar 27 from an image of the barbell exercise equipment photographed by the photographing unit 110. For example, certain patterns 28 may be arranged on the support bar 27 at certain intervals, and based on the patterns 28 of the support bar 27, an end length RL of the support bar 27, which is a part left unskewered after the weight plates 21A are skewered thereon, may be calculated. As the end length RL of the support bar 27 is a length calculated in correspondence with a distance between fixed patterns 28 and the number of the patterns 28, the end length RL may be calculated relatively accurately.

As such, the equipment detector 150 may detect the length of the support bar 27 from the exercise image photographed by the photographing unit 110, and the angle corrector 141 may correct, based on the length of the support bar 27, the rotation angle calculated by the angle calculator 132. For example, based on the length of the support bar 27, errors in the feature points C extracted by the feature point extractor 131 may be detected, and based on the detected errors in the feature points C, the rotation angle may be corrected.

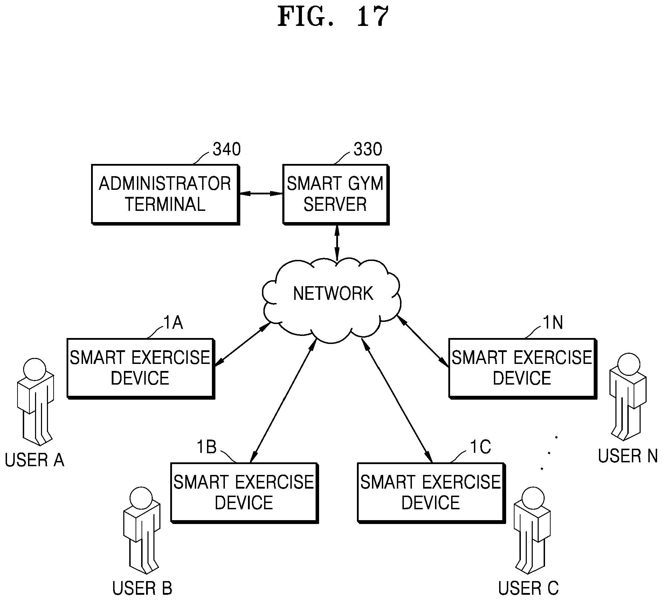

FIG. 17 is a diagram illustrating a smart gym environment in which a plurality of smart exercise devices are provided according to an embodiment.

Referring to FIG. 17, a plurality of smart exercise devices (1A, 1B, 1C, and 1N) may be connected to a smart gym server 200 through a network. An administrator such a trainer or a smart gym manger may access the smart gym server 200 through the administrator terminal 300.

Each of users (USER A, USER B, USER C, and USER N) who visit a smart gym may get into the smart gym after their identities are confirmed by using the terminal 320 such as a wearable device, a smartphone, etc. For example, the users may get into the gym after their identities are confirmed by tagging the terminal 320 to an unmanned terminal such as kiosk terminal installed at an entrance of the smart gym through near filed communication (NFC) or radio frequency identification (RFID). Information about the users whose identities are confirmed may be transmitted to at least one of the smart exercise devices (1A, 1B, 1C, and 1N) from the smart gym server 200 through a network.

When the user USER approaches at least one of the smart exercise devices (1A, 1B, 1C, and 1N) and tags a wearable device to the smart exercise device 1, the smart exercise device 1 may automatically set up an exercise program customized to an ability and an exercise performance history of the user USER based on information received from a smart gym server 330.

The smart gym server 330 may store user information of the plurality of users (USER A, USER B, USER C, and USER N), device information about the smart exercise devices (1A, 1B, 1C, and 1N), and information used for management of facilities or smart gym.

When an administrator such as a trainer register an exercise program customized to a user in the administrator terminal 300, exercise process information stored in the smart gym server 200 may be updated. The smart exercise devices (1A, 1B, 1C, and 1N) may receive the exercise process information from the smart gym server 200 connected thereto through a network. Although the leg extension for training the thigh muscles is described as an example of the exercise equipment of the smart exercise devices (1A, 1B, 1C, and 1N) in the embodiment above, the present disclosure is not limited thereto, and the smart exercise device may be applied to various other exercise equipment.

The embodiments according to the present disclosure described above may be implemented in the form of a computer program which can be executed in a computer through various components, and such computer program may be recorded on a computer-readable storage medium.

The exercise posture analyzing device may be implemented in a form of a computer-readable storage medium including at least one program for storing instructions executable by a processor. A computer refers to a device capable of calling a stored instruction from a storage medium and executing the operation described above according to a called instruction and may include the exercise posture analyzing device according to the embodiments described above. The computer-readable medium may include a magnetic medium, such as a hard disk, a floppy disk, and a magnetic tape, an optical recording medium, such as a CD-ROM and a DVD, a magneto-optical medium, such as a floptical disk, a hardware devices specially configured to store and execute program instructions, such as read-only memory (ROM), random-access memory (RAM), flash memory, etc. Moreover, the storage medium may include an intangible medium implemented in a form transmissible on a network, and may be, for example, a medium implemented in the form of software or application which may be transmitted and distributed through a network.

Meanwhile, the computer program may be specifically designed and configured for the present disclosure, or may be publicized for use to those skilled in the field of computer software. Not only machine language codes generated by a compiler, etc., but high level language codes which can be executed by a computer by using interpreters, etc. are also included in examples of the computer program.

So far, the present disclosure is described focusing on preferred embodiments. It will be understood by those skilled in the art that various changes in form and details may be made therein without departing from the spirit and scope of embodiments as defined by the present disclosure. Thus, the embodiments should be considered in a descriptive sense and not for purposes of limitation. The scope of the present disclosure is defined not by the detailed description of embodiments but by the appended claims, and all differences within the scope will be construed as being included in the present disclosure.

## Claims

1. A device for analyzing an exercise posture of a user using exercise equipment, the device comprising:
a photographing unit configured to obtain an exercise image of the user by photographing the user who is exercising by using the exercise equipment;
an equipment detector configured to detect exercise equipment-related information relating to at least one of a movement of the exercise equipment and a length of the exercise equipment; and
an analyzer configured to calculate a rotation angle of a body part of the user based on the exercise image and identify an exercise posture of the user based on the calculated rotation angle of the body part,
wherein the analyzer comprises:
a feature point extractor configured to extract a plurality of feature points corresponding a plurality of joints of the user based on the exercise image of the user;
an angle calculator configured to generate a plurality of bond lines corresponding to body parts of the user by connecting the plurality of feature points and calculate a rotation angle of at least one bond line of the plurality of bond lines;
an angle corrector configured to correct, based on the exercise equipment-related information, the rotation angle calculated by the angle calculator; and
a posture identifier configured to identify a degree of deviation of the rotation angle corrected by the angle corrector from a reference rotation angle to identify the exercise posture of the user.

2. The device of claim 1, wherein the photographing unit is a single camera installed to face a side of the exercise equipment.

3. The device of claim 1, wherein the exercise equipment is weight training equipment including a plurality of weight plates and an exercise structure delivering a load of the weight plates to the user, and
the equipment detector includes a first sensor configured to directly or indirectly detect a movement distance of the weight plates.

4. The device of claim 3, wherein the angle corrector is configured to:
calculate at least one of a movement distance and a rotation angle of the exercise structure based on the movement distance of the weight plates detected by the first sensor; and
correct, based on at least one of the movement distance and an angle change of the exercise structure, the rotation angle calculated by the angle calculator.

5. The device of claim 1, wherein the exercise equipment is barbell exercise equipment including a support bar and weight plates skewered on both ends of the support bar, and
the equipment detector is configured to obtain a length or a movement of the barbell exercise equipment.

6. The device of claim 5, wherein the equipment detector includes a second sensor installed in the barbell exercise equipment and configured to detect a movement of the barbell exercise equipment.

7. The device of claim 6, wherein the angle corrector is configured to detect a movement distance of the barbell exercise equipment based on a result detected by the second sensor, and
correct, based on the movement distance of the barbell exercise equipment, the rotation angle calculated by the angle calculator.

8. The device of claim 7, wherein the second sensor is arranged on the support bar.

9. The device of claim 5, wherein the photographing unit is configured to obtain an image of the support bar by photographing the support bar of the barbell exercise equipment, and
the angle corrector is configured to:
calculate a length of the support bar based on the image of the support bar; and
correct, based on the length of the support bar, the rotation angle calculated by the angel calculator.

10. A smart exercise device comprising exercise equipment and an exercise posture analyzing device configured to analyze an exercise posture of a user of the exercise equipment, wherein the exercise posture analyzing device comprises:
a photographing unit configured to obtain an exercise image of the user by photographing the user who is exercising by using the exercise equipment;
an equipment detector configured to detect exercise equipment-related information relating to at least one of a movement of the exercise equipment and a length of the exercise equipment; and
an analyzer configured to calculate a rotation angle of a body part of the user during exercise of the user based on the exercise image and identify an exercise posture of the user based on the calculated rotation angle of the body part, and
the analyzer comprises:
a feature point extractor configured to extract a plurality of feature points corresponding a plurality of joints of the user based on the exercise image of the user;
an angle calculator configured to generate a plurality of bond lines corresponding to body parts of the user by connecting the plurality of feature points and calculate a rotation angle of at least one bond line of the plurality of bond lines;
an angle corrector configured to correct, based on the exercise equipment-related information, the rotation angle calculated by the angle calculator; and
a posture identifier configured to identify a degree of deviation of the rotation angle corrected by the angle corrector from a reference rotation angle to identify the exercise posture of the user.

11. The smart exercise device of claim 10, wherein the photographing unit is a single camera installed to face a side of the exercise equipment.

12. The smart exercise device of claim 11, wherein the exercise equipment is weight training equipment including a plurality of weight plates and an exercise structure delivering a load of the weight plates to the user, and
the equipment detector includes a first sensor configured to directly or indirectly detect a movement distance of the weight plates.

13. The smart exercise device of claim 11, wherein the exercise equipment is barbell exercise equipment including a support bar and weight plates skewered on both ends of the support bar, and
the equipment detector is configured to obtain a length or a movement of the barbell exercise equipment.

14. The smart exercise device of claim 13, wherein the equipment detector includes a second sensor installed in the barbell exercise equipment and configured to detect a movement of the barbell exercise equipment.

15. A method of analyzing an exercise posture, the method comprising:
extracting a plurality of feature points corresponding a plurality of joints of a user based on an exercise image of the user obtained by a photographing unit;
generating a plurality of bond lines corresponding to body parts of the user by connecting the plurality of feature points and calculating a rotation angle of at least one bond line of the plurality of bond lines;
correcting the calculated rotation angle based on exercise equipment-related information detected by the equipment detector; and
identifying a degree of deviation of the corrected rotation angle from a reference rotation angle to identify an exercise posture of the user.
